(19) Europäisches Patentamt
European Patent Office
Office européen des brevets

(11) **EP 3 330 730 B1**

(12) **EUROPÄISCHE PATENTSCHRIFT**

(45) Veröffentlichungstag und Bekanntmachung des Hinweises auf die Patenterteilung:
**10.01.2024 Patentblatt 2024/02**

(21) Anmeldenummer: **17179796.2**

(22) Anmeldetag: **05.07.2017**

(51) Internationale Patentklassifikation (IPC):
***G01R 33/58*** *(2006.01)*

(52) Gemeinsame Patentklassifikation (CPC):
**G01R 33/586**

(54) **VERFAHREN ZUR OPTIMIERUNG EINES SIGNAL-RAUSCH-VERHÄLTNISSES EINES MAGNETRESONANZ(MR)-BILDES**

METHOD FOR OPTIMIZING A SIGNAL-TO-NOISE RATIO OF A MAGNETIC RESONANCE (MR) IMAGE

PROCÉDÉ D'OPTIMISATION D'UN RAPPORT SIGNAL/BRUIT D'UNE IMAGE PAR RÉSONANCE MAGNÉTIQUE (RM)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.06.2018 Patentblatt 2018/23**

(73) Patentinhaber: **Siemens Healthcare GmbH
91052 Erlangen (DE)**

(72) Erfinder:
• **Feiweier, Thorsten
91099 Poxdorf (DE)**
• **Hölscher, Uvo
91052 Erlangen (DE)**

(74) Vertreter: **Siemens Healthineers
Patent Attorneys
Siemens Healthcare GmbH
SHS TE IP
Henkestraße 127
91052 Erlangen (DE)**

(56) Entgegenhaltungen:
**US-A- 5 416 412**

• **BARTELS L W ET AL: "Improved Lumen Visualization in Metallic Vascular Implants by Reducing RF Artifacts", MAGNETIC RESONANCE IN MEDICINE, JOHN WILEY & SONS, INC, US, Bd. 47, Nr. 1, 1. Januar 2002 (2002-01-01), Seiten 171-180, XP002277498, ISSN: 0740-3194, DOI: 10.1002/MRM.10004**
• **DARDZINSKI B J ET AL: "Spatial variation of T2 in human articular cartilage", RADIOLOGY, RADIOLOGICAL SOCIETY OF NORTH AMERICA, INC, US, Bd. 205, Nr. 2, 1. Januar 1997 (1997-01-01), Seiten 546-550, XP002498044, ISSN: 0033-8419**

**Beschreibung**

Im Rahmen einer Magnetresonanzuntersuchung wird ein Patient auf einem Patiententisch positioniert, die für die Untersuchung bevorzugte Lokalspule, bspw. eine Kniespule oder eine Kopfspule, an der Magnetresonanzanlage angeschlossen und danach wird der Patient in die Bohrung der Magnetresonanzanlage verfahren. Daraufhin werden Justagemessungen unter anderem zur genauen Bestimmung der Resonanzfrequenz und zur Einstellung der Shimspulen vorgenommen.

Nach Durchführung aller Vorbereitungen können die eigentlichen Messungen durchgeführt werden. Hierzu werden Messprotokolle verwendet. Ein Messprotokoll ist eine Messsequenz mit voreingestellten Parametern, wobei die Voreinstellung für bestimmte Untersuchungen angepasst ist.

Mit typischen Messzeiten im Bereich weniger Minuten stellt die MR-Bildgebung nach wie vor ein relativ langsames, aber sehr wirksames Diagnosewerkzeug dar. Die Dauer der Messzeit wird letztlich durch die notwendige Aussagekraft der MR-Bilder bestimmt. Ein Ziel bei der MR-Bildgebung ist es, den gewünschten Kontrast in den MR-Bildern mit der benötigten Auflösung und einem hinreichend hohen Signal-zu-Rausch-Verhältnis (SNR) zu erzielen.

Entscheidend hierfür ist, dass das SNR über den gesamten für die Diagnose relevanten Aufnahmebereich mindestens einen Minimalwert erreicht. Der Minimalwert des SNR ist nicht notwendiger Weise für alle Regionen im Aufnahmebereich gleich groß. Vielmehr sollte das SNR in einem Bereich von Interesse, also einem kritischen Bereich hoch sein, während es in angrenzenden Regionen niedriger sein kann. Die Aufnahmedauer der MR-Bilder bestimmt sich folglich dadurch, dass in jedem relevanten Aufnahmebereich das notwenige Mindest-SNR erreicht wird.

Dieses kann beispielsweise durch zusätzliche Mittelungen geschehen. Gleichzeitig wird hierdurch in den angrenzenden Regionen die Bedingung des Mindest-SNR "übererfüllt".

Ein konkretes Beispiel für diesen Fall stellt beispielsweise die Diffusionsbildgebung im Kopf dar. Für klinische Magnetresonanzanlagen mit Magnetfeldstärken von bis zu 3T liefern derzeit eingesetzte Empfangsspulen-Konfigurationen im Hirnstamm ein deutlich geringeres SNR als in der Peripherie des Gehirns. Für bestimmte diagnostische Fragestellungen kann jedoch eine qualitativ hochwertige Darstellung des Hirnstammes, also die Aufnahme des Hirnstammes mit hohem SNR, bedeutend sein, während die an den Hirnstamm angrenzenden Regionen weniger bedeutend für die Diagnose sind und daher mit einem geringeren SNR gemessen werden könnten.

Die Erhöhung des SNR ist durch eine Vielzahl von Verfahren möglich. Beispielsweise kann durch Wiederholung der Bildaufnahme, also durch Signalmittelung, das SNR erhöht werden. Hierdurch verlängert sich allerdings die Messzeit. Ferner könnte auch die Repetitionszeit TR, also die Zeit, welche zwischen zwei aufeinander folgenden Anregungen derselben Schicht einer Region verstreicht, verlängert werden, um die verfügbare Longitudinalmagnetisierung für jede Signalanregung zu erhöhen. Auch hierbei kommt es zu einer Verlängerung der Messzeit und ferner zu Kontraständerungen. Es ist auch denkbar, das anliegende Magnetfeld zu erhöhen, wodurch jedoch die Kosten für den Betrieb einer Magnetresonanzanlage in die Höhe steigen. Eine Vergrößerung der Voxel, also eine geringere Auflösung zugunsten dickerer gemessener Schichten, führt zu unschärferen Bildern und zu Partial-Volumen Effekten.

Bei einer Aufnahme von MR-Bildern bzw. eines MR-Datensatzes während einer Magnetresonanzmessung besteht folglich das Problem, dass durch die Erhöhung des SNR über sämtliche zu erfassenden Regionen sich die Messzeit verlängert und dadurch manche Regionen mit einem übererfülltem SNR gemessen werden.

XP002277498 (Bartels et al.; Improved Lumen Visualization in Metallic Vascular Implants by Reducing RF Artifacts; Magnetic Resonance in Medicine 47:171-180 (2002)) betrifft einen Artikel über die Verbesserung der Lumen-Visualisierung in metallischen Gefäßimplantaten, wie Stents und Vena Cava Filtern, durch Reduzierung von RF-Artefakten. Ein Ziel war die Erforschung, ob auftretende RF-Artefakte in metallischen Gefäßimplantaten durch eine Erhöhung der angewendeten RF-Leistung korrigiert werden können. Hierbei wurde herausgefunden, dass ein reduziertes Signal in den metallischen Gefäßimplantaten nur beobachtbar ist, wenn es eine Kopplung zwischen dem Gefäßimplantat und der Empfangsspule gibt.

US 5,416,412 A betrifft ein Verfahren und eine Vorrichtung zum schnellen Messen des Nutationswinkels oder Kippwinkels, der durch ein Anregungsfeld in einer MRI-Anlage erzeugt wird, und insbesondere ein Verfahren und eine Vorrichtung zum Einstellen des HF-Leistungspegels für eine MRI-Anlage, bevor eine Abtastung erfolgt. Die Lehre der US 5, 416 412 A umfasst das Anlegen von drei Anregungs-Feldimpulsen in einem interessierenden Bereich zusammmen mit einem magnetischen Schicht-Auswahl-Feldgradienten, das Gewinnen von vier resultierenden MR-Echosignalen in Anwesenheit eines orthogonalen, magnetischen Auslese-Feldgradienten, die Fourier-Transformation der vier MR-Echosignale, die Berechnung der Amplitudenwerte aus den vier transformierten MR-Echosignalen sowie die Berechnung des Nutationswinkels, der von dem ersten Anregungs-Feldimpuls unter Benutzung der Amplitudenwerte aus den jeweiligen MR-Echosignalen erzeugt wird.

XP002498044 (Dardzinski et al.: "Spatial Variation of T2 in Human Articular Cartilage", Radiology, Radiological Society of North America, Inc. US, Bd. 205, Nr. 2 (1. November 1997)) betrifft einen Artikel über die räumliche Variation von T2 im menschlichen Gelenkknorpel. Hierin ist offenbart, dass zu Beginn einer jeden Studie die 90° und 180° abschnittsselektiven Impulse durch Maximierung der Signalintensität optimiert werden.

Es ist Aufgabe der vorliegenden Erfindung, ein Verfahren zum Betrieb einer Magnetresonanzanlage anzugeben, das ein erhöhtes SNR in Regionen von Interesse aufweist, ohne einen der oben genannten Nachteile, insbesondere ohne eine Verlängerung der Messzeit oder ein Verlust an Auflösung.

Diese Aufgabe wird erfindungsgemäß mit einem Verfahren zum Betrieb einer Magnetresonanzanlage gemäß Anspruch 1 gelöst. Vorteilhafte Weiterbildungen der Erfindung sind Gegenstand der abhängigen Ansprüche.

Als Kern der Erfindung wird angesehen, dass eine Transmitter-Skalierung in mindestens einer Region von Interesse derart optimiert wird,, insbesondere bevor die eigentliche MR-Messung durchgeführt wird, dass in der mindestens einen Region von Interesse ein Kriterium für ein Signal-Rausch-Verhältnis (SNR) erfüllt wird. Ein Kriterium für ein Signal-Rausch-Verhältnis (SNR) beinhaltet beispielsweise, dass das SNR in einem Bereich eines lokalen Minimums angehoben wird, ohne dass das SNR in anderen Bereichen unter diesen angehobenen Wert abfällt. Es wird folglich ein Verfahren zur Optimierung eines Signal-Rausch-Verhältnisses (SNR) eines Magnetresonanz(MR)-Datensatzes gemäß Anspruch 1 vorgeschlagen, welcher mit einer Magnetresonanzanlage mit wenigstens einer Empfangsspule akquiriert wird, mit den Schritten:

- Bereitstellen eines Messprotokolls für eine durchzuführende Akquisition eines Magnetresonanz(MR)-Datensatzes von einem vorgegebenen Messvolumen, wobei das Messprotokoll zumindest einen Hochfrequenzpuls mit einem vorgegebenen Flipwinkel umfasst, welcher für die Signalamplitude relevant ist, und wobei es sich bei dem Messvolumen um eine Schicht, mehrere Schichten oder ein Volumen, d.h. ein slab, einer 3D-Messung handelt;
- Bestimmen einer Abweichung eines tatsächlichen Flipwinkels von dem vorgegebenen Flipwinkel in einem bestimmten Bereich des vorgegebenen Messvolumens bei einer voreingestellten Transmitter-Skalierung;
- Anpassen der Transmitter-Skalierung des Hochfrequenzpulses zum Korrigieren des tatsächlichen Flipwinkels, so dass in dem bestimmten Bereich der tatsächliche Flipwinkel an den vorgegebenen Flipwinkel angenähert wird, und so, dass das Signal-Rausch-Verhältnis (SNR) in dem gesamten vorgegebenen Messvolumen über einem Mindestwert liegt, und
- anschließende Akquisition des Magnetresonanz(MR)-Datensatzes mit der angepassten Transmitter-Skalierung.

Insbesondere können gemäß dem vorgeschlagenen Verfahren mehrere Regionen von Interesse festgelegt werden, in welchen der tatsächliche Flipwinkel bestimmt und angepasst wird. Ferner wird in der oder in den Regionen von Interesse eine EmpfangsSensitivität der Empfangsspule bestimmt. Nach einer Auswertung der Abweichung des vorgegebenen Flipwinkels von dem tatsächlichen Flipwinkel und der Empfangs-Sensitivität in einer Region von Interesse wird die Transmitter-Skalierung derart optimiert, dass in der Region von Interesse ein SNR-Kriterium erfüllt wird. Die Bestimmung der Abweichung des vorgegebenen Flipwinkels von dem tatsächlichen Flipwinkel entspricht einer Abschätzung des Einflusses des Flipwinkels auf das SNR für ein vorgegebenes Messprotokoll. Das SNR wird folglich insbesondere durch den Flipwinkel, durch die Empfangs-Sensitivität und durch das Messprotokoll bestimmt.

Erfindungsgemäß handelt es sich bei dem Messvolumen um eine Schicht, mehrere Schichten oder ein Volumen, d.h. ein slab, einer 3D-Messung. Der bestimmte Bereich ist bevorzugt ein Bereich innerhalb einer Schicht bzw. eines Volumens, kann aber auch eine von mehreren Schichten sein.

Mit dem Begriff "Transmitter-Skalierung" ist eine Einstellung des Flipwinkels durch eine entsprechende Ansteuerung der Sendespule gemeint, mit der die Signalamplitude bzw. der Signalpegel der für die MR-Messung relevanten Hochfrequenzpulse angesteuert bzw. erhöht oder erniedrigt wird, je nachdem ob der Flipwinkel erhöht oder reduziert werden soll. Die Transmitter-Skalierung ist relevant für die Auslenkung einer vorliegenden Longitudinalmagnetisierung. Die Transmitter-Skalierung wird üblicherweise automatisch von der Steuereinrichtung der Magnetresonanzanlage derart eingestellt, dass über ein Messvolumen "im Mittel" der vorgegebene Flipwinkel erreicht wird. Vorschlagsgemäß wird die Transmitter-Skalierung in dem Messvolumen von Interesse angepasst, so dass ein vorgegebenes SNR vor allem in der Region von Interesse, wie beispielsweise dem Hirnstamm, erreicht wird.

Zur Bestimmung der Abweichung des tatsächlichen Flipwinkels von dem vorgegebenen Flipwinkel in einem bestimmten Bereich des vorgegebenen Messvolumens bei einer voreingestellten Transmitter-Skalierung werden Messverfahren genutzt, bei denen eine tatsächliche räumliche Verteilung der erreichten Flipwinkel für eine voreingestellte Transmitter-Skalierung direkt gemessen wird. Eine derart erfasste ortsaufgelöste Flipwinkel-Karte liefert direkt eine Abweichung des tatsächlichen Flipwinkels von dem vorgegebenen Flipwinkel. Ein solches Messverfahren ist beispielsweise in der DE 103 38 074 B4 beschrieben. Die am Ort x empfangene MR-Signalamplitude S(x) und damit folglich das SNR skaliert mit dem räumlichen Sensitivitätsprofil R(x) der Empfangsspule und dem Feldprofil der Sendespule im Objekt T(x), also dem zu untersuchenden Patienten, gemäß

$$S(x) \sim R(x) * f(\alpha(x)),$$

wobei $\alpha(x)$ der lokale tatsächliche Flipwinkel ist und durch

$$\alpha(x) = \gamma \int T(x) \, B_1(t) \, dt$$

gegeben ist. Die Konstante $\gamma$ beschreibt das gyromagnetische Verhältnis und $B_1(t)$ den zeitlichen HF-Pulsverlauf. Die Funktion $f(\alpha(x))$ gibt an, wie sich für ein bestimmtes Messverfahren oder ein bestimmtes Messprotokoll der Flipwinkel $\alpha(x)$ in eine Signal-generierende Transversalmagnetisierung übersetzt. So findet man beispielsweise für ein Gradientenecho-Experiment mit TR >> $T_1$, dass $f_{SE}(\alpha) \sim \sin(\alpha)$ gilt, und für ein Spin-Echo-Experiment ($\alpha$-2$\alpha$-Echo) mit TR>> $T_1$, dass $f_{SE}(\alpha) - \sin^3(\alpha)$ gilt, und für ein Doppel-Spin-Echo-Experiment ($\alpha$-2$\alpha$-2$\alpha$-Echo) entsprechend, dass $f_{SE}(\alpha) \sim \sin^5(\alpha)$ gilt. Ferner sind komplexere Abhängigkeiten unter dem Begriff "Steady-State der Magnetisierung" bei einer kürzeren Repetitionszeit TR bekannt. Das Sensitivitätsprofil R(x) von Empfangsspulen, insbesondere von hochkanaligen Varianten und bei einer Feldstärke von mindestens 3T, ist im Zentrum des Kopfes (x=0) deutlich geringer als in der Peripherie, also dem Rand bei(x=R). Dies ist ein Grund, warum das SNR bei konventionellen MR-Messungen im Hirnstamm geringer ist als in der Peripherie. Die Anpassung des tatsächlichen Flipwinkels an den vorgegebenen Flipwinkel in den Bereichen von Interesse gemäß dem vorgeschlagenen Verfahren hat folglich den Vorteil, dass die Erfassung des MR-Datensatzes mit einem homogenisierten SNR durgeführt werden kann. Hierdurch wird in den Regionen von Interesse ein Mindestmaß des SNR erreicht und damit sind die Regionen von Interesse besser darstellbar, so dass das resultierende MR-Bild eine höhere Aussagekraft, insbesondere bessere Kontraste, zeigt. Abhängig davon, ob die angepasste Transmitter-Skalierung den Flipwinkel oder die Flipwinkel erhöht oder reduziert, fällt die HF-Belastung des Patienten höher oder niedriger aus. Insbesondere bei der 3T-Kopfbildgebung sinkt in der Regel die HF-Belastung des Patienten, so dass als positiver Effekt schnellere Aufnahmen, insbesondere durch Reduktion der Repetitionszeit TR, der MR-Messdaten möglich werden.

Bevorzugt liegt der bestimmte Bereich in einer Region minimaler Sensitivität der Empfangsspule, insbesondere im Bereich der geringsten Sensitivität der Empfangsspule. Durch Optimierung in dieser Region wird somit das Signal in diesem Bereich gesteigert; in anderen Bereichen ist der Flipwinkel hingegen durch die Transmitter-Skalierung verschlechtert worden, wodurch das Signal abnimmt. Durch die Verteilung der Sensitivität der Empfangsspule wird dieser Effekt beim Empfang des Signals zumindest teilweise ausgeglichen: Das hohe Signal aus dem bestimmten Bereich wird mit der geringsten Sensitivität empfangen. Dadurch wird das Signal-zu-Rausch-Verhältnis über das gesamte Messvolumen homogenisiert.

Die Abweichung des tatsächlichen Flipwinkels von dem vorgegebenen Flipwinkel wird in vorteilhaften Ausführungsformen durch eine Messung bestimmt. Zur Bestimmung der Abweichung des tatsächlichen Flipwinkels von dem vorgegebenen Flipwinkel in einem bestimmten Bereich des vorgegebenen Messvolumens bei einer voreingestellten Transmitter-Skalierung können Messverfahren genutzt werden, bei denen eine tatsächliche räumliche Verteilung der erreichten Flipwinkel für eine voreingestellte Transmitter-Skalierung direkt gemessen wird. Eine derart erfasste, insbesondere ortsaufgelöste, Flipwinkel-Karte liefert direkt eine Abweichung des tatsächlichen Flipwinkels von dem vorgegebenen Flipwinkel. Ein solches Messverfahren ist beispielsweise in der DE 103 38 074 B4 beschrieben. Die Bestimmung der Abweichung beeinflusst die Anpassung der Transmitter-Skalierung.

In vorteilhaften Ausführungsformen wird die Abweichung des tatsächlichen Flipwinkels von dem vorgegebenen Flipwinkel aufgrund von Erfahrungswerten bestimmt, welche für die Akquisition von Magnetresonanz(MR)-Datensätzen einer bestimmten Körperregion in Kombination mit einer bestimmten Sendespule in einer Datenbank hinterlegt sind. Hierzu werden die erfassten Signale, welche für eine bestimmte Sendspule bei einem angelegten Hochfrequenzpuls mit bestimmten Sendepegel bzw. mit bestimmter Transmitter-Skalierung, und ggf. mit einer bestimmten Empfangsspule, erfasst wurden, mit den Parametern der Sendespule und dem angelegten Magnetfeld korreliert. Bei der Korrelation dieser Werte wird mittels bekannter Optimierungsverfahren, welche beispielsweise einen "Simplex Downhill"- oder eines "Levenberg-Marquardt"- Algorithmus oder dergleichen verwenden, die Abweichung bestimmt. Bei diesen Verfahren wird eine Kostenfunktion bestimmt und dann eine Minimierung der Kostenfunktion durchgeführt.

Die Transmitter-Skalierung des Hochfrequenzpulses wird erfindungsgemäß derart angepasst, dass das Signal-Rausch-Verhältnis (SNR) in dem gesamten vorgegebenen Messvolumen über einem Mindestwert liegt. Indem die Transmitter-Skalierung des Hochfrequenzpulses derart justiert wird, dass das Signal-Rausch-Verhältnis (SNR) in dem gesamten vorgegebenen Messvolumen über einem Mindestwert liegt, wird das Signal-Rausch-Verhältnis (SNR) über dem gesamten Messvolumen durch Erreichen eines Mindest-SNR homogenisiert, so dass über das gesamte Messvolumen ein für die Diagnose aussagekräftiges Messsignal erfasst werden kann.

Bevorzugt wird zur Homogenisierung des Signal-Rausch-Verhältnisses über das vorgegebene Messvolumen das Signal-Rausch-Verhältnis in Bereichen mit einem hohen SNR, insbesondere in Bereichen der Peripherie, der zu untersuchenden Region des Objektes reduziert und/oder in Bereichen mit einem geringen SNR, insbesondere in einem zentralen Bereich, der zu untersuchenden Region des Objektes erhöht. Das SNR ist somit über die zu untersuchende Region des Objektes homogenisiert. Bevorzugt werden zum Anpassen der Transmitter-Skalierung Erfahrungswerte verwendet, welche für die Akquisition von Magnetresonanz(MR)-Datensatzes einer bestimmten Körperregion in Kombination mit einer bestimmten Empfangsspule in einer Datenbank hinterlegt sind. Besonders bevorzugt sind die Erfahrungswerte einer bestimmten Körperregion in Kombination mit einer bestimmten Empfangsspule und/oder einer bestimmten Sendespule bei einem vorgegebenen Magnetfeld hinterlegt.

Ferner bevorzugt erfolgt die Anpassung der Transmitter-Skalierung auf Basis von zumindest einer der folgenden Informationen:

- eines Sensitivitätsprofils der Empfangsspule, welches bekannt ist oder durch Messung bestimmt wird,
- einer räumlichen Verteilung der Abweichung des tatsächlichen vom vorgegeben Flipwinkel im vorgegebenen Messvolumen,
- der Abhängigkeit des relativen Signalpegels vom Flipwinkel.

Das Sensitivitätsprofil der Empfangsspule kann in einer Datenbank hinterlegt sein oder vor der Durchführung einer MR-Messung erfasst werden. Bei Verwendung einer räumlichen Verteilung der Abweichung des tatsächlichen vom vorgegeben Flipwinkel im vorgegebenen Messvolumen können in einer Datenbank hinterlegte Erfahrungswerte herangezogen werden. Es ist auch denkbar, die räumliche Verteilung aktuell zu messen und für die anstehende MR-Messung zu verwenden. Hierzu wird in unterschiedlichen Schichten, insbesondere der gesamten zu erfassenden Region, ortsaufgelöst eine Abweichung vom tatsächlichen Flipwinkel gemessen. Ferner ist es denkbar, eine Abhängigkeit des relativen Signalpegels vom Flipwinkel zu erfassen oder solche Erfahrungswerte aus einer Datenbank zu beziehen. Hierzu wird der tatsächliche Flipwinkel mit dem dazugehörigen Signalpegel korreliert. Es ist ferner denkbar eine oder mehrere dieser Informationen zur Anpassung der Transmitter-Skalierung heranzuziehen. Hierdurch wird die Anpassung der Transmitter-Skalierung insgesamt optimiert.

Bevorzugt liegen alle genannten Informationen vor und die Transmitter-Skalierung wird durch ein Optimierungsverfahren derart bestimmt, dass der zu erwartende relative Signalpegel an keinem Ort im vorgegebenen Messvolumen einen vorgegebenen Minimalwert unterschreitet. Bevorzugte Optimierungsverfahren sind Verfahren zur Optimierung nichtlinearer Funktionen wie beispielsweise das Simplex-Downhill-Verfahren oder das Levenberg-Marquardt-Verfahren. Der vorgegebene Minimalwert entspricht einem Mindest-SNR, welches bestimmt wird durch den Flipwinkel, durch die Empfangssensitivität und/oder durch das Messprotokoll. Insbesondere liegt der Minimalwert des SNR bei mindestens 5, bevorzugt bei 10 und besonders bevorzugt bei 20.

Das Verfahren wird bevorzugt für alle Hochfrequenzpulse eines Messprotokolls angewendet, die für die Signalamplitude relevant sind, also insbesondere Anregungs- und Refokussierungspulse. Bevorzugt werden zum Erreichen eines vorbestimmten Kontrastes oder einer Unterdrückung bestimmter Signalbeiträge zusätzliche Pulse verwendet, für welche keine Transmitter-Skalierung vorgenommen wird, insbesondere wird ein Inversions-Puls (IR), und/oder ein chemisch-selektiver Sättigungspuls (CSat), und/oder ein räumlich selektiver Unterdrückungspuls (RSat) verwendet.

Bevorzugt umfasst das vorgegebene Messvolumen mehrere Schichten und die Transmitter-Skalierung wird unabhängig für jede Schicht vorgenommen. Das erfindungsgemäße Verfahren kann vorteilhaft mit Techniken kombiniert werden, bei denen die Transmitter-Skalierung individuell für jede Schicht angepasst werden kann ("dynamische Justagen" bzw. "Slice Adjust"). Dabei werden die zuvor beschriebenen Optimierungen der Transmitter-Skalierung für jedes Subvolumen (einzelne Schicht bzw. einzelner Slab) vorgenommen.

Bevorzugt sieht das vorgegebene Messprotokoll ein Schicht-Multiplexing-Verfahren vor. Dabei werden Sub-Pulse einer simultanen Anregung und die bei einer Refokussierung erfassten Sub-Volumina einzeln erfasst und betrachtet. Jeder Sub-Puls kann dann beispielsweise unabhängig optimiert bzw. skaliert werden. Es ist ferner denkbar, das vorgeschlagene Verfahren mit anderen Techniken zu kombinieren, bei denen die Transmitter-Skalierung individuell für jede Schicht angepasst werden kann. Hierbei werden die Optimierungen der Transmitter-Skalierung für jedes Sub-Volumen, bzw. für jede Schicht, vorgenommen.

In einigen Ausführungsformen wird der bestimmte Bereich automatisch ausgewählt. Der bestimmte Bereich kann in einem Messprotokoll, welches zur Durchführung der Untersuchung mittels MR-Bildgebung ausgesucht wurde, hinterlegt sein. Es ist ebenfalls denkbar, den bestimmten Bereich bei Bedarf für eine angehende Untersuchung auch manuell zu bestimmen oder manuell zu vergrößern oder zu verkleinern. Es ist ferner denkbar, mehrere bestimmte Bereiche zu definieren. Hierdurch wird insbesondere in Regionen von großem Interesse ein erhöhtes SNR erreicht, während in den jeweils angrenzenden Regionen ein geringeres SNR erreicht wird.

Ferner bevorzugt ist der bestimmte Bereich durch einen Benutzer auswählbar. Es können unterschiedliche vorgegebene vorbestimmte Bereiche in einem ausgesuchten Messprotokoll hinterlegt sein. Ferner ist es denkbar, den ausgesuchten bestimmten Bereich bei Bedarf für eine angehende Untersuchung manuell durch den Benutzer zu vergrößern oder zu verkleinern.

Ein weiterer Aspekt der vorliegenden Erfindung betrifft eine Magnetresonanzanlage, welche wenigstens eine Steuereinrichtung umfasst, wobei die Steuerungseinrichtung zum Durchführen eines der zuvor beschriebenen Verfahren ausgebildet ist.

Bevorzugt weist die Magnetresonanzanlage eine Eingabeeinrichtung auf, welche einem Anwender Auswahlmöglichkeiten der bestimmten Region eines Objektes, insbesondere die Auswahlmöglichkeit "optimiere Hirnstamm", oder grafische Auswahlmöglichkeiten anzeigt. Bei einer grafischen Auswahlmöglichkeit kann beispielsweise ein Untersuchungsobjekt, wie beispielsweise ein Mensch, schematisch auf einem Bildschirm angezeigt werden, wobei unterschiedliche Regionen für die Untersuchung, insbesondere visuell, vorgeschlagen werden. Dies hat den Vorteil, dass ein Anwender schnell ein vorgegebenes Messprotokoll auswählen kann, ohne dass der Anwender besonders mit der Magnetresonanzanlage vertraut sein muss.

Weitere vorteilhafte Ausgestaltungen des erfindungsgemäßen Verfahrens korrespondieren zu entsprechenden Ausgestaltungen der erfindungsgemäßen Magnetresonanzanlage. Zur Vermeidung unnötiger Wiederholungen wird somit auf die entsprechenden Verfahrensmerkmale und deren Vorteile verwiesen.

Gemäß einem weiteren Aspekt der vorliegenden Erfindung wird ein Computerprogramm vorgeschlagen, das ein zuvor beschriebenes Verfahren auf einer zuvor beschrieben Steuereinrichtung einer Magnetresonanzanlage implementiert, wenn es auf der Steuereinrichtung ausgeführt wird. Die Implementierung der vorgenannten Verfahren in der Steuervorrichtung kann dabei als Software oder aber auch als, insbesondere fest verdrahtete, Hardware erfolgen.

Gemäß einem weiteren Aspekt der vorliegenden Erfindung wird ein elektronisch lesbarer Datenträger mit darauf gespeicherten elektronisch lesbaren Steuerinformationen vorgeschlagen, welche zumindest ein zuvor beschriebenes Computerprogramm umfassen und derart ausgestaltet sind, dass sie bei Verwendung des Datenträgers in einer Steuereinrichtung einer Magnetresonanzanlage ein hierin beschriebenes Verfahren durchführen.

Das vorgeschlagene Verfahren, die vorgeschlagene Magnetresonanzanlage, das vorgeschlagene Computerprogramm und der vorgeschlagene elektronisch lesbare Datenträger sind nicht auf die Kopfbildgebung eingeschränkt. Vielmehr finden sie Anwendung in allen Situationen, bei denen sich am Ort der minimalen Empfangsspulen-Sensitivtät mit einem lokal optimierten, angepassten Flipwinkel die Signalamplitude erhöhen lässt.

Weitere Vorteile, Merkmale und Besonderheiten der vorliegenden Erfindung ergeben sich aus der nachfolgenden Beschreibung vorteilhafter Ausgestaltungen der Erfindung.

Dabei zeigen

Fig. 1 eine Magnetresonanzanlage,
Fig. 2 Ablaufschema des vorgeschlagenen Verfahrens, und
Fig. 3 MR-Bild eines Kopfes in Höhe des Hirnstammes, welches konventionell aufgenommen wurde und
Fig. 4 MR-Bild eines Kopfes in Höhe des Hirnstammes, welches mit dem vorgeschlagenen Verfahren aufgenommen wurde.

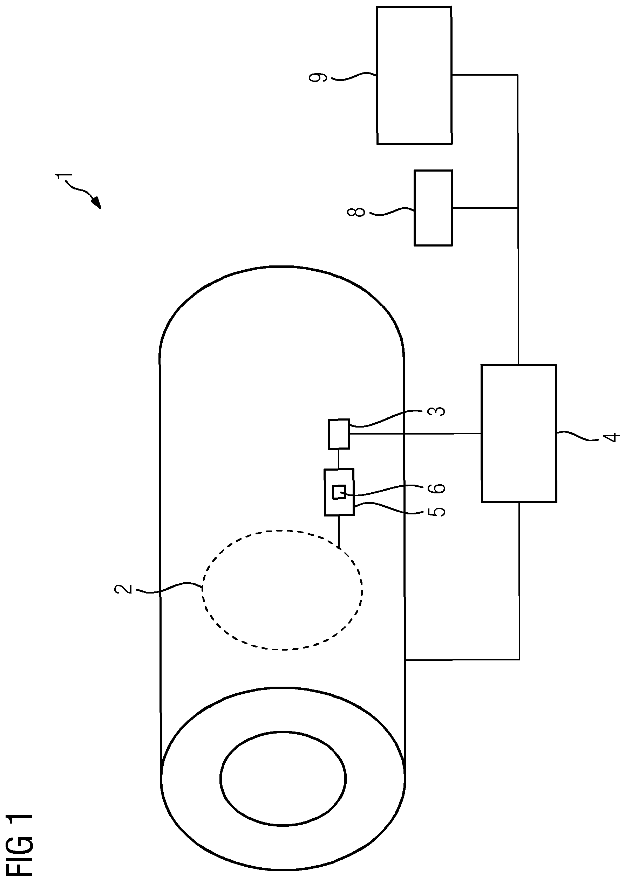

Figur 1 zeigt eine Magnetresonanzanlage 1 mit wenigstens einer Empfangs- und/oder Sendespule 2, nämliche einer Hochfrequenzspule 2. Die Hochfrequenzspule 2 ist über einen Anschluss 3 mit der Steuerungseinrichtung 4 der Magnetresonanzanlage 1 verbunden, wobei der Anschluss 3 eine elektrische Verbindung ermöglicht. Der Anschluss 3 kann als Steckverbindung ausgestaltet sein. Insbesondere ermöglicht der Anschluss 3, verschiedene Hochfrequenzspulen 2 mit der Steuerungseinrichtung zu verbinden. Diese Darstellung ist selbstverständlich vereinfacht, zwischen der Hochfrequenzspule 2 und der Steuerungseinrichtung 4 der Magnetresonanzanlage befinden sich üblicherweise mehr Elemente als der Anschluss 3. Der Anschluss 3 erlaubt den Austausch von unterschiedlichen Hochfrequenzspulen 2.

Die Hochfrequenzspule 2 besitzt einen Informationsträger 5, der einen Informationscode 6 trägt. Dieser Informationscode 6 ist eine Spulenidentifikationsinformation. Bspw. besteht der Identifikationscode 6 aus einer Ziffernfolge wie den Ziffern 124, welche z. B. für eine bestimmte Kopfspule stehen.

Weiterhin sind eine Anzeigevorrichtung 8 und eine Eingabeeinrichtung 9 mit der Steuerungseinrichtung 4 verbunden. Auf der Anzeigevorrichtung 8 können unterschiedliche bestimmte Bereiche 36 grafisch angezeigt werden.

Das beschriebene Verfahren ist bevorzugt als Software, also als ein Computerprogramm, in der Steuerungseinrichtung 4 implementiert. Ein hierin beschriebenes Verfahren kann damit in Form eines Computerprogrammprodukts vorliegen, das ein hierin beschriebenes Verfahren auf der der Steuerungseinrichtung 4 implementiert, wenn es auf der Steuerungseinrichtung 4 ausgeführt wird. Ebenso kann ein elektronisch lesbarer Datenträger (nicht dargestellt) mit darauf gespeicherten elektronisch lesbaren Steuerinformationen vorliegen, welche zumindest das beschriebene Computerprogramm umfassen und derart ausgebildet sind, dass sie bei Verwendung des Datenträgers in der Steuerungseinrichtung 4 der Magnetresonanzanlage 1 ein hierin beschriebenes Verfahren durchführen.

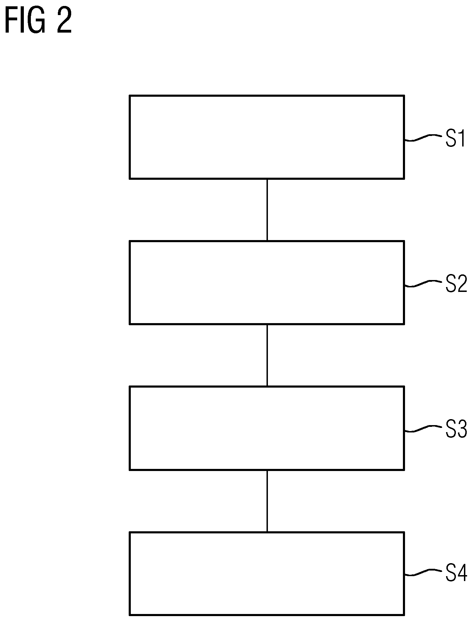

Figur 2 zeigt ein Ablaufschema des vorgeschlagenen Verfahrens zum Betrieb der Magnetresonanzanlage 1 mit den Schritten S1 bis S4.

Im ersten Schritt S1 wird ein Messprotokoll für eine durchzuführende Akquisition eines Magnetresonanz(MR)-Datensatzes von einem vorgegebenen Messvolumen bereitgestellt, wobei das Messprotokoll zumindest einen Hochfrequenzpuls mit einem vorgegebenen Flipwinkel $\alpha_{soll}$ umfasst, welcher für die Signalamplitude $S(x)$ relevant ist.

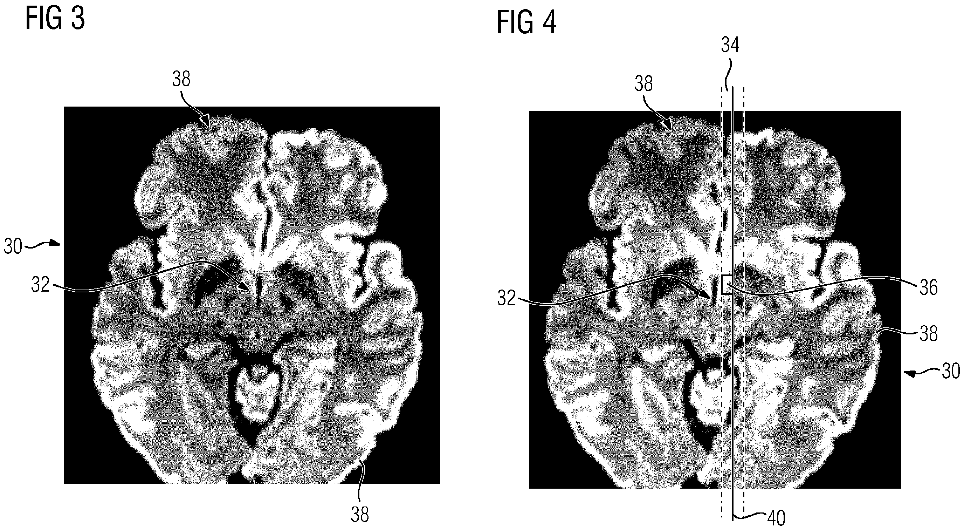

Im zweiten Schritt S2 wird eine Abweichung eines tatsächlichen Flipwinkels $\alpha(x)$ von dem vorgegebenen Flipwinkel $\alpha_{soll}$ in einem bestimmten Bereich 36 des vorgegebenen Messvolumens, welches gemäß Figur 4 als Schicht 30 gegeben ist, bei einer voreingestellten Transmitter-Skalierung bestimmt.

Im dritten Schritt S3 wird die Transmitter-Skalierung des Hochfrequenzpulses zum Korrigieren des tatsächlichen Flipwinkels $\alpha(x)$ angepasst, so dass in dem bestimmten Bereich der tatsächliche Flipwinkel $\alpha(x)$ an den vorgegebenen Flipwinkel $\alpha_{soll}$ angenähert wird. Die Bestimmung einer optimierten Transmitter-Skalierung in einer zu untersuchenden Schicht 30 der Region kann beispielsweise empirisch erfolgen. Hierfür können Erfahrungswerte, welche einer bestimmten Körperregion zugeordnet sind, wie beispielsweise dem Kopf oder dem Knie, in Kombination mit einer bestimmten Empfangsspule 2 und/oder einer bestimmten Sendespule 2, wie beispielsweise eine 64-Kanal-Kopfspule, in einer Datenbank hinterlegt sein. Die Hochfrequenzspule 2 kann als eine Sendespule 2 oder als eine Empfangsspule 2 oder als eine als Sende- und Empfangsspule agierende Spule ausgebildet sein.

Anschließend wird im vierten Schritt S4 die Akquisition eines Magnetresonanz(MR)-Datensatzes mit der angepassten Transmitter-Skalierung durchgeführt.

Figur 3 zeigt ein konventionell aufgenommenes MR-Bild und Figur 4 zeigt ein mittels des vorgeschlagenen Verfahrens aufgenommenes axiales MR-Diffusionsbild einer Schicht 30 eines Kopfes eines Patienten auf Höhe des Hirnstammes 32 im Vergleich.

Beide diffusionsgewichteten Bilder der Figuren 3 und 4 wurden mit einer relativ hohen Auflösung bei einem anliegenden Magnetfeld von 3T und mit einer 64-Kanal-Kopfspule aufgenommen. Bei einem verwendeten Magnetfeld von 3T ist der vorgegebene Flipwinkel von beispielsweise $\alpha=90°$ für den Anregungspuls bei konventionellen MR-Messungen nur im räumlichen Mittel über den gesamten Kopf erreicht. Im Zentrum des Kopfes bei x=0 weichen die tatsächlichen Flipwinkel beispielsweise bei konventionellen Verfahren um 10% bis 15% von dem vorgegebenen Flipwinkel ab. Hierdurch wird bei konventionellen Verfahren nur ein reduziertes SNR erreicht.

Figur 3 zeigt ein konventionell aufgenommenes MR-Bild, bei dem die Transmitter-Skalierung vom System derart eingestellt wird, dass im Mittel der vorgegebene Flipwinkel $\alpha_{soll}$ erreicht wird. Im Hirnstamm 32 ist das Signal-Rausch-Verhältnis SNR geringer als in der Peripherie 38, da die verwendete Empfangsspule eine geringe Sensitivität R(x) im Bereich des Hirnstammes 32 aufweist.

Insbesondere wenn - wie im Fall der Kopfbildgebung bei 3T mit Mehrkanal-Empfangsspulen - die Region der

1) minimalen Empfangsspulensensitivität gleichzeitig einen für die Signalbetrachtung
2) suboptimalen Flipwinkel aufweist, greift das erfindungsgemäße Verfahren:

1. Bestimmung der Abweichung des tatsächlichen lokalen Flipwinkels vom nominalen Wert für diese Region.

2. Anpassung der Transmitter-Skalierung der für die Signalamplitude relevanten HF-Pulse derart, dass lokal der Nominalwert erreicht wird.
3. Durchführung der Messung.

Auf diese Weise erreicht man eine Homogenisierung des SNR:

- In der Region mit der geringsten Empfangsspulensensitivität wird das SNR erhöht (beispielsweise im Hirnstamm)
- In Regionen mit höherer Empfangsspulensensitivität wird das SNR ggf. reduziert (beispielsweise in der Peripherie des Kopfes)

Insbesondere bei Spin-Echo-Messungen entspricht der nominale Flipwinkel einem SNR-optimalen Wert. Bei anderen Messungen hingegen weist der nominale Flipwinkel nicht notwendigerweise ein maximales SNR auf. Vielmehr kann der Flipwinkel auch derart eingestellt sein, dass ein optimaler Kontrast erreicht wird.

Figur 4 zeigt im Bereich des Hirnstammes 32 ein erhöhtes SNR. Bei der Akquisition der Figur 4 wurde die Transmitter-Skalierung derart angepasst, dass der vorgegebene Flipwinkel im Zentrum des Kopfes, also im Hirnstamm 32, erreicht wurde. Hierdurch wurde insbesondere in einem Teilbereich 34 der Schicht 30 lokal der Nominalwert erreicht ist. Wie dargestellt umfasst der Teilbereich 34 den bestimmten Bereich 36. Das Bezugszeichen 40 kennzeichnet eine Mittelpunktlinie entlang dem Teilbereich 34. Hierdurch wurde somit das SNR im Bereich des Hirnstammes 32 deutlich erhöht. Gleichzeitig ist in der Peripherie 38 ein hinreichendes SNR, erfindungsgemäß das Mindest-SNR, erhalten geblieben, obgleich sich in dem Bereich der Peripherie 38 der Flipwinkel $\alpha$ durch die Transmitter-Skalierung noch weiter vom vorgegebenen Flipwinkel entfernt hat. Das ausreichend hohe SNR ist auf die dort hohe Empfangsspulensensitivität zurückzuführen.

**Patentansprüche**

1. Verfahren zur Optimierung eines Signal-Rausch-Verhältnisses (SNR) eines Magnetresonanz(MR)-Datensatzes, welcher mit einer Magnetresonanzanlage (1) mit wenigstens einer Empfangsspule (2) akquiriert wird, mit den Schritten:

    - Bereitstellen eines Messprotokolls für eine durchzuführende Akquisition eines Magnetresonanz(MR)-Datensatzes von einem vorgegebenen Messvolumen, wobei das Messprotokoll zumindest einen Hochfrequenzpuls mit einem vorgegebenen Flipwinkel umfasst, welcher für die Signalamplitude relevant ist, und wobei es sich bei dem Messvolumen um eine Schicht, mehrere Schichten oder ein Volumen, d.h. ein slab, einer 3D-Messung handelt;

- Bestimmen einer Abweichung eines tatsächlichen Flipwinkels von dem vorgegebenen Flipwinkel in einem bestimmten Bereich (36) des vorgegebenen Messvolumens bei einer voreingestellten Transmitter-Skalierung;

- Anpassen der Transmitter-Skalierung des Hochfrequenzpulses zum Korrigieren des tatsächlichen Flipwinkels, so dass in dem bestimmten Bereich (36) der tatsächliche Flipwinkel an den vorgegebenen Flipwinkel angenähert wird und so, dass das Signal-Rausch-Verhältnis (SNR) in dem gesamten vorgegebenen Messvolumen über einem Mindestwert liegt, und

- anschließende Akquisition des Magnetresonanz(MR)-Datensatzes mit der angepassten Transmitter-Skalierung.

2. Verfahren nach Anspruch 1, **dadurch gekennzeichnet, dass** der bestimmte Bereich (36) in einer Region minimaler Sensitivität der Empfangsspule (2) liegt, insbesondere im Bereich der geringsten Sensitivität der Empfangsspule (2) .

3. Verfahren nach Anspruch 1 oder 2, **dadurch gekennzeichnet, dass** die Abweichung des tatsächlichen Flipwinkels von dem vorgegebenen Flipwinkel durch eine Messung bestimmt wird.

4. Verfahren nach einem der vorhergehenden Ansprüche, **dadurch gekennzeichnet, dass** die Abweichung des tatsächlichen Flipwinkels von dem vorgegebenen Flipwinkel aufgrund von Erfahrungswerten bestimmt wird, welche für die Akquisition von Magnetresonanz(MR)-Datensätzen einer bestimmten Körperregion in Kombination mit einer bestimmten Sendespule (2) in einer Datenbank hinterlegt sind.

5. Verfahren nach einem der vorhergehenden Ansprüche, **dadurch gekennzeichnet, dass** zur Homogenisierung des Signal-Rausch-Verhältnisses (SNR) über das vorgegebene Messvolumen das Signal-Rausch-Verhältnis in Bereichen mit einem hohen SNR, insbesondere in Bereichen der Peripherie (38), der zu untersuchenden Region des Objektes reduziert und/oder in Bereichen mit einem geringen SNR, insbesondere in einem zentralen Bereich, der zu untersuchenden Region des Objektes erhöht wird.

6. Verfahren nach einem der vorhergehenden Ansprüche, **dadurch gekennzeichnet, dass** zum Anpassen der Transmitter-Skalierung Erfahrungswerte verwendet werden, welche für die Akquisition von Magnetresonanz(MR)-Datensatzes einer bestimmten Körperregion in Kombination mit einer bestimmten Empfangsspule (2) in einer Datenbank hinterlegt sind.

7. Verfahren nach einem der vorhergehenden Ansprüche, **dadurch gekennzeichnet, dass** die Anpassung der Transmitter-Skalierung auf Basis von zumindest einer der folgenden Informationen erfolgt:

- eines Sensitivitätsprofils der Empfangsspule (2), welches bekannt ist oder durch Messung bestimmt wird,
- einer räumlichen Verteilung der Abweichung des tatsächlichen vom vorgegebenen Flipwinkel im vorgegebenen Messvolumen,
- der Abhängigkeit des relativen Signalpegels vom Flipwinkel.

8. Verfahren nach Anspruch 7, **dadurch gekennzeichnet, dass** alle genannten Informationen vorliegen und die Transmitterskalierung durch ein Optimierungsverfahren derart bestimmt wird, dass der zu erwartenden relative Signalpegel an keinem Ort im vorgegebenen Messvolumen einen vorgegebenen Minimalwert unterschreitet.

9. Verfahren nach einem der vorhergehenden Ansprüche, **dadurch gekennzeichnet, dass** das vorgegebene Messvolumen mehrere Schichten (30) umfasst und die Transmitter-Skalierung unabhängig für jede Schicht (30) vorgenommen wird.

10. Verfahren nach einem der vorhergehenden Ansprüche, **dadurch gekennzeichnet, dass** das vorgegebene Messprotokoll ein Schicht-Multiplexing-Verfahren vorsieht.

11. Verfahren nach einem der vorhergehenden Ansprüche, **dadurch gekennzeichnet, dass** der bestimmte Bereich (36) automatisch ausgewählt wird.

12. Verfahren nach einem der vorhergehenden Ansprüche, **dadurch gekennzeichnet, dass** der bestimmte Bereich (36) durch einen Benutzer auswählbar ist.

13. Magnetresonanzanlage (1) umfassend wenigstens eine Steuereinrichtung (4), **dadurch gekennzeichnet, dass** die Steuerungseinrichtung (4) zum Durchführen eines Verfahrens nach einem der vorhergehenden Ansprüche ausgebildet ist.

14. Magnetresonanzanlage (1) nach Anspruch 13, **dadurch gekennzeichnet, dass** eine Eingabeeinrichtung (9) vorgesehen ist, welche einem Anwender Auswahlmöglichkeiten der bestimmten Region eines Objektes, insbesondere die Auswahlmöglichkeit "optimiere Hirnstamm", oder grafische Auswahlmöglichkeiten anzeigt.

15. Computerprogramm, das ein Verfahren nach einem der Ansprüche 1 bis 12 auf einer Steuereinrichtung (4) einer Magnetresonanzanlage (1) implementiert, wenn es auf der Steuereinrichtung (4) ausgeführt wird.

16. Elektronisch lesbarer Datenträger mit darauf gespeicherten elektronisch lesbaren Steuerinformationen, welche zumindest ein Computerprogramm nach Anspruch 15 umfassen und derart ausgestaltet sind, dass sie bei Verwendung des Datenträgers in einer Steuereinrichtung (4) einer Magnetresonanzanlage (1) das Verfahren nach einem der Ansprüche 1-12 durchführen.

**Claims**

1. Method for optimising a signal-to-noise ratio (SNR) of a magnetic resonance (MR) dataset which is acquired by means of a magnetic resonance system (1) having at least one receive coil (2), said method comprising the steps:

   - providing a measurement protocol for an acquisition that is to be performed in order to obtain a magnetic resonance (MR) dataset of a predefined measurement volume, wherein the measurement protocol comprises at least one radiofrequency pulse having a predefined flip angle that is relevant to the signal amplitude, and wherein the measurement volume is a slice, a plurality of slices or a volume, i.e. a slab, of a 3D measurement;
   - determining a deviation of an actual flip angle from the predefined flip angle in a specific area (36) of the predefined measurement volume for a preset transmitter scaling;
   - adjusting the transmitter scaling of the radiofrequency pulse in order to correct the actual flip angle so that the actual flip angle is approximated to the predefined flip angle in the specific area (36), and so that the signal-to-noise ratio (SNR) lies above a minimum value in the entire predefined measurement volume; and
   - subsequent acquisition of the magnetic resonance (MR) dataset at the adjusted transmitter scaling.

2. Method according to claim 1, **characterised in that** the specific area (36) is located in a region of minimum sensitivity of the receive coil (2), in particular in the area of the lowest sensitivity of the receive coil (2).

3. Method according to claim 1 or 2, **characterised in that** the deviation of the actual flip angle from the predefined flip angle is determined by means of a measurement.

4. Method according to one of the preceding claims, **characterised in that** the deviation of the actual flip angle from the predefined flip angle is determined on the basis of empirical values which are stored in a database for the acquisition of magnetic resonance (MR) datasets of a specific body region in combination with a specific transmit coil (2).

5. Method according to one of the preceding claims, **characterised in that** in order to homogenise the signal-to-noise ratio (SNR) over the predefined measurement volume, the signal-to-noise ratio is reduced in areas of the region to be examined of the object that have a high SNR, in particular in areas of the periphery (38), and/or is increased in areas of the region to be examined of the object that have a low SNR, in particular in a central area.

6. Method according to one of the preceding claims, **characterised in that** in order to adjust the transmitter scaling, empirical values are used which are stored in a database for the acquisition of a magnetic resonance (MR) dataset of a specific body region in combination with a specific receive coil (2).

7. Method according to one of the preceding claims, **characterised in that** the transmitter scaling is adjusted on the basis of at least one of the following pieces of information:

   - a sensitivity profile of the receive coil (2) which is known or is determined by measurement,
   - a spatial distribution of the deviation of the actual from the predefined flip angle in the predefined measurement volume,
   - the dependence of the relative signal level on the flip angle.

8. Method according to claim 7, **characterised in that** all of the cited information is available and the transmitter scaling is determined by means of an optimisation method in such a way that the expected relative signal level does not fall below a predefined minimum value at any location in the predefined measurement volume.

9. Method according to one of the preceding claims, **characterised in that** the predefined measurement volume comprises a plurality of slices (30) and the transmitter scaling is performed independently for each slice (30).

10. Method according to one of the preceding claims, **characterised in that** the predefined measurement protocol provides a slice multiplexing method.

11. Method according to one of the preceding claims, **characterised in that** the specific area (36) is selected automatically.

**12.** Method according to one of the preceding claims, **characterised in that** the specific area (36) may be selected by a user.

**13.** Magnetic resonance system (1) comprising at least one control device (4), **characterised in that** the control device (4) is embodied to perform a method according to one of the preceding claims.

**14.** Magnetic resonance system (1) according to claim 13, **characterised in that** an input device (9) is provided which displays selection options for choosing the specific region of an object to a user, in particular the selection option "optimise brain stem", or graphical selection options.

**15.** Computer program which implements a method according to one of claims 1 to 12 on a control device (4) of a magnetic resonance system (1) when the computer program is executed on the control device (4).

**16.** Electronically readable data medium on which there is stored electronically readable control information comprising at least a computer program according to claim 15 and embodied in such a way that it performs the method according to one of claims 1-12 when the data medium is used in a control device (4) of a magnetic resonance system (1).

**Revendications**

**1.** Procédé d'optimisation d'un rapport (SNR) signal - bruit, d'un ensemble de données de résonance magnétique (RM), que l'on acquiert avec une installation (1) de résonance magnétique par au moins une bobine (2) de réception, comprenant les stades :

- on se procure un protocole de mesure pour une acquisition à effectuer d'un ensemble de données de résonance magnétique (RM) d'un volume de mesure donné à l'avance, dans lequel le protocole de mesure comprend au moins une impulsion en haute fréquence ayant un angle de flip donné à l'avance, qui est pertinent pour l'amplitude du signal, et dans lequel le volume de mesure est une couche, plusieurs couches ou un volume, c'est-à-dire un slab, d'une mesure en 3D ;
- on détermine un écart d'un angle de flip réel à l'angle de flip donné à l'avance dans une partie (36) déterminée du volume de mesure donné à l'avance pour une mise à l'échelle de transmetteur réglée à l'avance ;
- on adapte la mise à l'échelle de transmetteur de l'impulsion en haute fréquence pour la correction de l'angle de flip réel de sorte que, dans la partie (36) déterminée, l'angle de flip réel se rapproche de l'angle de flip donné à l'avance, et de sorte que le rapport (SNR) signal - bruit dans tout le volume de mesure donné à l'avance est supérieur à une valeur minimum, et
- on acquiert ensuite l'ensemble de données de résonnance magnétique (RM) avec la mise à l'échelle de transmetteur adapté.

**2.** Procédé suivant la revendication 1, **caractérisé en ce que** la partie (36) déterminée se trouve dans une région de sensibilité minimum de la bobine (2) de réception, en particulier dans la partie de sensibilité la plus petite de la bobine (2) de réception.

**3.** Procédé suivant la revendication 1 ou 2, **caractérisé en ce que** l'on détermine, par une mesure, l'écart de l'angle de flip réel à l'angle de flip donné à l'avance.

**4.** Procédé suivant l'une des revendications précédentes, **caractérisé en ce que** l'on détermine l'écart de l'angle de flip réel à l'angle de flip donné à l'avance, sur la base de valeurs expérimentales, qui sont mises dans une base de données, pour l'acquisition d'ensembles de données de résonnance magnétique (RM) d'une région déterminée du corps, en combinaison avec une bobine (2) d'émission déterminée.

**5.** Procédé suivant l'une des revendications précédentes, **caractérisé en ce que**, pour l'homogénéisation du rapport (SNR) signal - bruit sur le volume de mesure donné à l'avance, on réduit le rapport signal - bruit dans des parties ayant un grand SNR, en particulier dans des parties de la périphérie (38), de la région à examiner de l'objet et/ou on l'augmente dans des parties ayant un SNR petit, en particulier dans une partie centrale de la région à examiner de l'objet.

**6.** Procédé suivant l'une des revendications précédentes, **caractérisé en ce que**, pour l'adaptation de la mise à l'échelle de transmetteur, on utilise des valeurs expérimentales, qui sont mises dans une base de données pour l'acquisition d'ensembles de données de résonnance magnétique (RM) d'une région du corps déterminée en combinaison avec une bobine (2) de réception déterminée.

**7.** Procédé suivant l'une des revendications précédentes, **caractérisé en ce que** l'adaptation de la mise à l'échelle de transmetteur s'effectue sur la base d'au moins l'une des informations suivantes :

- un profil de sensibilité de la bobine (2) de réception, qui est connu ou que l'on détermine par mesure,

- une répartition dans l'espace de l'écart de l'angle de flip réel à l'angle de flip donné à l'avance dans le volume de mesure donné à l'avance,
- la variation du niveau relatif de signal de l'angle de flip.

**8.** Procédé suivant la revendication 7, **caractérisé en ce que** toutes les informations mentionnées sont présentes et on détermine la mise à l'échelle de transmetteur par un procédé d'optimisation, de manière à ce que le niveau relatif de signal à escompter ne soit inférieur en aucun emplacement du volume de mesure donné à l'avance à une valeur minimum donnée à l'avance.

**9.** Procédé suivant l'une des revendications précédentes, **caractérisé en ce que** le volume de mesure donné à l'avance comprend plusieurs couches (30) et on a effectué la mise à l'échelle de transmetteur indépendamment pour chaque couche (30) .

**10.** Procédé suivant l'une des revendications précédentes, **caractérisé en ce que** le protocole de mesure donné à l'avance prévoit un procédé de multiplexage de couche.

**11.** Procédé suivant l'une des revendications précédentes, **caractérisé en ce que** l'on choisit automatiquement la partie (36) déterminée.

**12.** Procédé suivant l'une des revendications précédentes, **caractérisé en ce que** la partie (36) déterminée peut être choisie par un utilisateur.

**13.** Installation (1) de résonnance magnétique comprenant au moins un dispositif (4) de commande, **caractérisée en ce que** le dispositif (4) de commande est constitué pour effectuer un procédé suivant l'une des revendications précédentes.

**14.** Installation (1) de résonnance magnétique suivant la revendication 13, **caractérisée en ce qu'**il est prévu un dispositif (9) d'entrée, qui indique à un utilisateur des possibilités de sélection de la région déterminée d'un objet, en particulier la possibilité de sélection « tronc cérébral optimisé » ou des possibilités de sélection graphique.

**15.** Programme d'ordinateur, qui met en œuvre un procédé suivant l'une des revendications 1 à 12 sur un dispositif (4) de commande d'une installation (1) de résonnance magnétique, lorsqu'il est réalisé sur le dispositif (4) de commande.

**16.** Support de données, déchiffrable électroniquement, sur lequel sont mises en mémoire des informations de commande déchiffrables électroniquement, qui comprennent au moins un programme d'ordinateur suivant la revendication 15, et qui sont conformées de manière à ce que, lorsque le support de données est utilisé dans un dispositif (4) de commande d'une installation (1) de résonnance magnétique, elles effectuent le procédé suivant l'une des revendications 1 à 12.

FIG 1

EP 3 330 730 B1

FIG 2

FIG 3

FIG 4

EP 3 330 730 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5416412 A

- DE 10338074 B4

**In der Beschreibung aufgeführte Nicht-Patentliteratur**

- **BARTELS et al.** Improved Lumen Visualization in Metallic Vascular Implants by Reducing RF Artifacts. *Magnetic Resonance in Medicine,* 2002, vol. 47, 171-180

- Spatial Variation of T2 in Human Articular Cartilage. **DARDZINSKI et al.** Radiology. Radiological Society of North America, Inc, 01. November 1997, vol. 205